# EUROPÄISCHE PATENTSCHRIFT

(11) **EP 1 381 669 B1**
(45) Veröffentlichungstag und Bekanntmachung des Hinweises auf die Patenterteilung: **11.01.2006**
(21) Anmeldenummer: 02737810.8
(22) Anmeldetag: 23.04.2002
(51) Int. Cl.: C12M 1/34, C12M 1/36

(54) **VERFAHREN UND VORRICHTUNG ZUR UNTERSUCHUNG VON FERMENTATIONSPROZESSEN**
METHOD AND DEVICE FOR INVESTIGATION OF FERMENTATION PROCESSES
PROCEDE ET DISPOSITIF D'ANALYSE DE PROCESSUS DE FERMENTATION

(30) Priorität: 27.04.2001 DE 10120839
(43) Veröffentlichungstag der Anmeldung: 21.01.2004
(73) Patentinhaber: FORSCHUNGSZENTRUM JÜLICH GMBH, 52425 Jülich (DE)
(72) Erfinder: EL MASSAOUDI, Mohamed, 52428 Jülich (DE); FRANZ, Andreas, 50389 Wesseling (DE); DE GRAAF, Albert, A., NL-6418 CB Heerlen (NL); TAKORS, Ralf, 52399 Merzenich (DE)
(86) Internationale Anmeldenummer: PCT/DE2002/001478
(87) Internationale Veröffentlichungsnummer: WO 2002/088297

(56) Entgegenhaltungen:
- EP-A- 0 422 876
- DE-C- 4 334 677
- US-A- 4 798 798

## Beschreibung

Die Erfindung betrifft ein Verfahren sowie eine Vorrichtung zur Untersuchung von Fermentationsprozessen.

Zur Untersuchung von Fermentationsprozessen sind verschiedene Verfahren bekannt. Bei der Untersuchung des metabolischen Stoffflusses wird aus den extrazellulär bestimmten Verbrauchs- bzw. Produktbildungsraten durch Bilanzierung auf intrazelluläre Stoffflüsse geschlossen (Joergensen J., Nielsen J., Villadsen J.: "Metabolic flux distributions in *Penicillium chrysogenum* during fed-batch cultivations"; Biotechnol. Bioeng. (1995); 46, 117-131). Es lassen sich jedoch nicht die gesamten Stoffflüsse des Zentralstoffwechsels bestimmen, da Bilanzierungslücken auftreten und Annahmen wie z. B. über den Energiehaushalt der Zelle gemacht werden müssen.

Weiterhin sind Verfahren bekannt, die über eine Markierung des Zentralstoffwechsels mit z. B. ¹³C-Glukose und anschließender Analyse des Biomassehydrolysats mit Hilfe von z. B. Kernmagnetischer Resonanzspektroskopie (NMR) den intrazellulären Stofffluß sehr detailliert quantifizieren (Wiechert W.: "Metabolische Kohlenstoff-Markierungssysteme- Modellierung, Simulation, Analyse, Datenauswertung" (1995); Habilitationsschrift Universität Bonn). Diese Methode ist jedoch nur in kontinuierlich betriebenen Fermentationsverfahren optimal anwendbar. Die in kontinuierlicher Kultur gewonnenen Markierungsdaten können nicht auf großtechnische Fermentationsprozesse übertragen werden, da diese Fermentationsprozesse bis auf wenige Ausnahmen hauptsächlich im Satzbetrieb (Batch) oder im Satzbetrieb mit kontinuierlicher Medienzudosierung (Fedbatch) betrieben werden. Für einen Markierungsversuch im großtechnischen Maßstab (z. B. 100 m³) stellen die hohen Kosten der Markierungssubstanzen, wie z. B. ¹³C-Glukose (ca. 300 DM/g) ein erhebliches Problem dar.
Ein weiterer Nachteil der bisher gängigen, detaillierten metabolischen Stoffflussanalyse mit Hilfe der Markierungsmethode ist, dass sich nach der Zugabe der Markierungssubstanz die Markierung in der gesamten Zellmasse anreichert. So lassen sich innerhalb eines Versuches keine Mehrfachmarkierungen durchführen. Für jedes neue Markierungsexperiment muss eine erneute Fermentation durchgeführt werden. Einzelne Abschnitte einer Fermentation können so nicht untersucht werden. Generell werden Untersuchungen für großtechnische Fermentationsprozesse zunächst im kleineren Labormaßstab durchgeführt. Die Ergebnisse, die im Labormaßstab gewonnen werden, sind jedoch auf Grund unterschiedlicher Ausgangsbedingungen der eingesetzten Zellen nur bedingt auf den Fermentationsprozess im großtechnischen Produktionsreaktor übertragbar. Direkte Untersuchungen des Fermentationsprozesses im technischen Maßstab sind sehr kosten- und arbeitsaufwendig.

Aus der europäischen Patentanmeldung 0 422 876 A1 ist ein Verfahren und eine Vorrichtung zur Kontrolle von Abwasseraufarbeitung mittels anaerober Fermentation bekannt.

Es ist daher die Aufgabe der Erfindung ein Verfahren sowie eine Vorrichtung zu schaffen, mit der Untersuchungen von technischen bzw. großtechnischen Fermentationsprozessen möglich werden ohne die laufende Fermentation zu beeinflussen.

Ausgehend vom Oberbegriff des Anspruchs 1 wird die Aufgabe erfindungsgemäß gelöst mit den im kennzeichnenden Teil des Anspruchs 1 angegebenen Merkmalen. Weiterhin wird die Aufgabe ausgehend vom Oberbegriff des Anspruchs 20 erfindungsgemäß gelöst mit den im kennzeichnenden Teil des Anspruchs 20 angegebenen Merkmalen.

Mit dem erfindungsgemäßen Verfahren und der Vorrichtung ist es nunmehr möglich im kleinen Maßstab mehrfach Kontrollfermentationen synchron zum großtechnischen Produktionsreaktor durchzuführen und die daraus gewonnenen Ergebnisse mit hoher Korrelation auf den Produktionsreaktor zu übertragen. Mit der erfindungsgemäßen Vorrichtung und dem Verfahren ist es weiterhin möglich, ohne Beeinflussung der laufenden Fermentation metabolische Stoffflussanalysen zu beliebig wählbaren Zeitpunkten durchzuführen und gleichzeitig die dabei entstehenden Markierungskosten zu minimieren.

Vorteilhafte Weiterbildungen sind in den Unteransprüchen angegeben.

Die Zeichnungen zeigen eine beispielhafte Ausführungsform des erfindungsgemäßen Verfahrens und der Vorrichtung sowie beispielhaft experimentelle Ergebnisse des Verfahrens und der Vorrichtung. Es zeigt:
- Fig. 1:: Schematische Anordnung der Vorrichtung
- Fig. 2:: Korrelation der Biomassebildung im Sensor Reaktor und Produktionsreaktor
- Fig. 3:: Korrelation der L-Lysin Konzentration im Sensor Reaktor und Produktiönsreaktor
- Fig. 4:: Korrelation der L-Leucin Konzentration im Sensor Reaktor und Produktionsreaktor
- Fig. 5:: Korrelation der Glycin Konzentration im Sensor Reaktor und Produktionsreaktor
- Fig. 6:: Korrelation der Oxoglutarat Konzentration im Sensor Reaktor und Produktionsreaktor

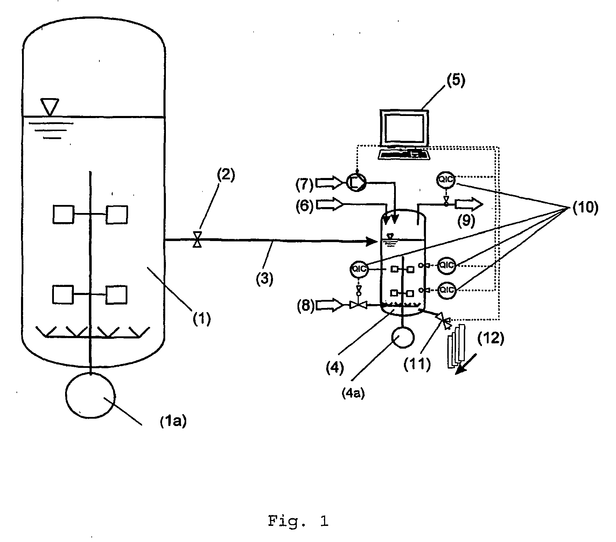

Figur 1 zeigt einen Produktionsreaktor 1 mit motorbetriebenem Rührer 1a, der über eine Leitung 3 mit Ventilen 2 mit einem Sensor Reaktor 4 mit motorbetriebenem Rührer 4a verbunden ist. Die Vorrichtung umfaßt eine Prozessüberwachung 5. An den Sensor Reaktor 4 sind Zugabesysteme 6 und 7 zur Zufuhr von Substanzen sowie ein Zugabesystem 8 zur Zufuhr von Gas angebracht. Weiterhin umfaßt der Sensor Reaktor 4 ein Abfuhrsystem 9 zur Entnahme der Abluft. An den Sensor Reaktor 4 sind verschiedene Meß-/Regelsysteme 10 angeschlossen. Der Sensor Reaktor verfügt außerdem über eine Leitung mit Probenahmeventil 11 die in eine Probenahmeeinheit 12 mündet.

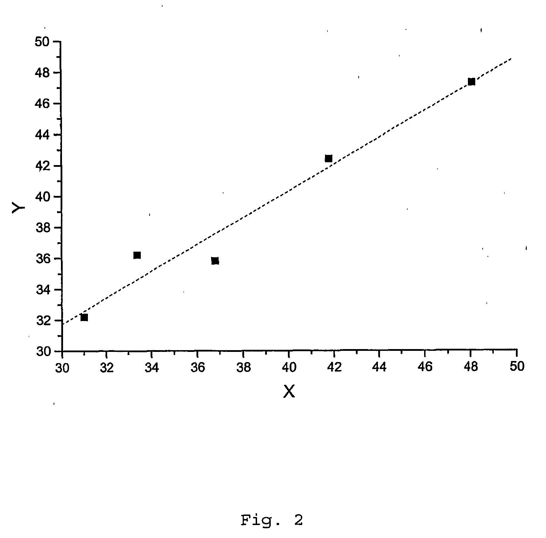

Figur 2 zeigt den Vergleich der Biomassekonzentration während einer Fermentation in einem 300 l Produktionsreaktor und einem 1 l Sensor Reaktor. Gemessen wurden die optischen Dichten der Proben aus beiden Reaktoren bei einer Wellenlänge von 600 nm. Dabei gibt die Abszisse X die optische Dichte im 300 l Produktionsreaktor und die Ordinate Y die optische Dichte im 1 l Sensor Reaktor an. Die mit ■ markierten Punkte geben das Verhältnis der optischen Dichte im Sensor Reaktor zum Produktionsreaktor an. Die gestrichelte Gerade stellt die Regressionsgerade dar. Der Korrelationskoeffizient R beträgt 0,97911.

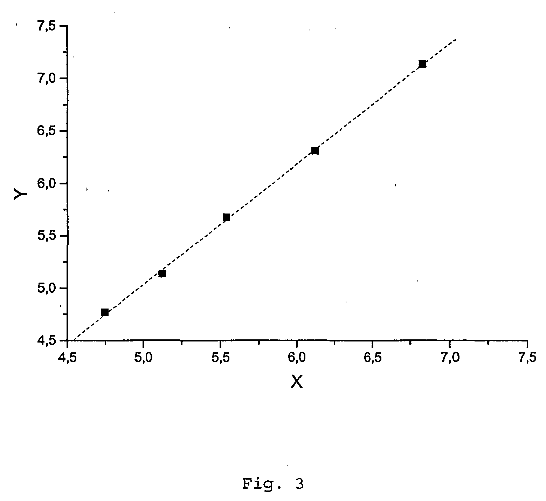

Figur 3 zeigt den Vergleich der L-Lysinkonzentration während einer Fermentation in einem 300 l Produktionsreaktor und einem 1 l Sensor Reaktor. Gemessen wurde die L-Lysinkonzentration im Medienüberstand der Proben aus beiden Reaktoren mit Hilfe einer HPLC. Dabei gibt die Abszisse X die L-Lysinkonzentration im 300 l Produktionsreaktor und die Ordinate Y die L-Lysinkonzentration im 1 l Sensor Reaktor an. Die mit ■ markierten Punkte geben das Verhältnis der L-Lysinkonzentration im Sensor Reaktor zum Produktionsreaktor an. Die gestrichelte Gerade stellt die Regressionsgerade dar. Der Korrelationskoeffizient R beträgt 0,99957.

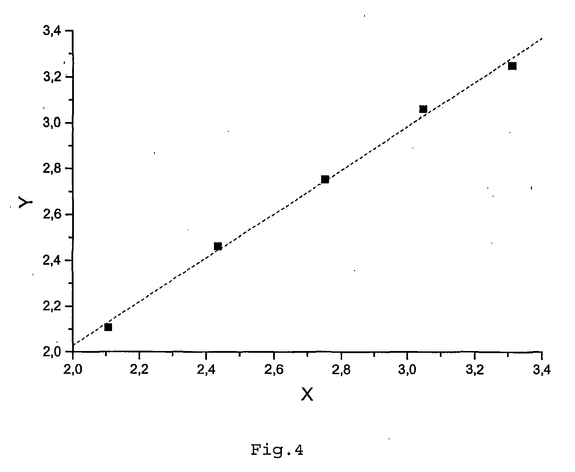

Figur 4 zeigt den Vergleich der L-Leucinkonzentration während einer Fermentation in einem 300 l Produktionsreaktor und einem 1 l Sensor Reaktor. Gemessen wurden die L-Leucinkonzentration im Medienüberstand der Proben aus den Reaktoren mit Hilfe einer HPLC. Dabei gibt die Abszisse x die L-Leucinkonzentration im 300 l Produktionsreaktor und die Ordinate Y die L-Leucinkonzentration im 1 l Sensor Reaktor an. Die mit ■ markierten Punkte geben das Verhältnis der L-Leucinkonzentration im Sensor Reaktor zum Produktionsreaktor an. Die gestrichelte Gerade stellt die Regressionsgerades dar. Der Korrelationskoeffizient R beträgt 0,99826.

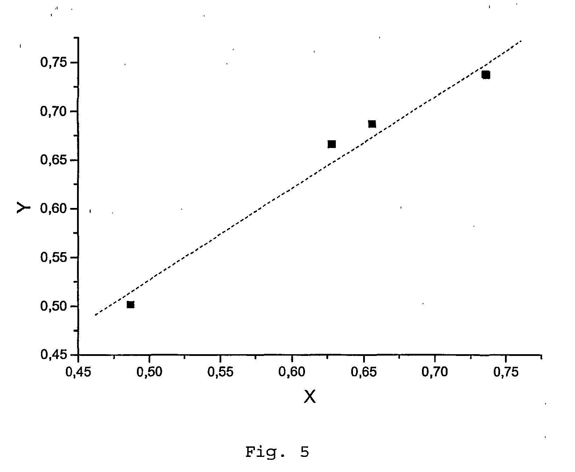

Figur 5 zeigt den Vergleich der Glycinkonzentration während einer Fermentation in einem 300 l Produktionsreaktor und einem 1 l Sensor Reaktor. Gemessen wurde die Glyeinkonzentration im Medienüberstand der Proben aus den Reaktoren mit Hilfe einer HPLC. Dabei gibt die Abszisse X die Glycinkonzentration im 300 l Produktionsreaktor und die Ordinate Y die Glycinkonzentration im 1 l Sensor Reaktor an. Die mit ■ markierten Punkte geben das Verhältnis der Glycinkonzentration im Sensor Reaktor zum Produktionsreaktor an. Die gestrichelte Gerade stellt die Regressionsgerade dar. Der Korrelationskoeffizient R beträgt 0,98747.

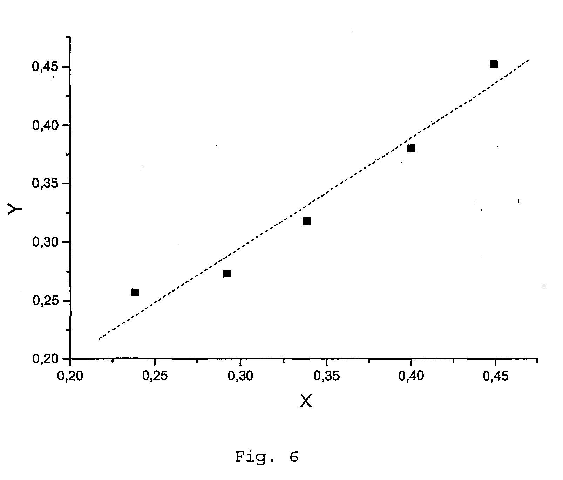

Figur 6 zeigt den Vergleich der Oxoglutaratkonzentration während einer Fermentation in einem 300 l Produktionsreaktor und einem 1 l Sensor Reaktor. Gemessen wurden die Oxoglutaratkonzentration im Medienüberstand der Proben aus den Reaktoren mit Hilfe einer HPLC. Dabei gibt die Abszisse X die Oxoglutaratkonzentration im 300 l Produktionsreaktor und die Ordinate Y die Oxoglutaratkonzentration im 1 l Sensor Reaktor an. Die mit ■ markierten Punkte geben das Verhältnis der Oxoglutaratkonzentration im Sensor Reaktor zum Produktionsreaktor an. Die gestrichelte Gerade stellt die Regressionsgerade dar. Der Korrelationskoeffizient R beträgt 0,97756.

Im folgenden soll die Erfindung beispielhaft beschrieben werden.

Zur Untersuchung des Fermentationsprozesses eines gerührten Produktionsreaktors 1 wird ein gerührter kleinerer Reaktor, im folgenden als Sensor Reaktor 4 bezeichnet, über eine Leitung 3 an den Produktionsreaktor 1 angeschlossen. Der Sensor Reaktor 4 muß nicht die gleiche Reaktorgeometrie wie der Produktionsreaktor 1 aufweisen. Eine ähnliche Reaktorgeometrie ist ausreichend. Die Reaktorgröße und der Reaktortyp sind je nach Anforderung beliebig variierbar. Als Sensor Reaktoren 4 können beispielsweise Reaktoren mit einem Volumen von 0,5 bis 10 l eingesetzt werden. Demgegenüber kann das Volumen des Produktionsreaktors 1 maximal übliche Industriereaktoren (ca. 500 m³) umfassen und besonders bevorzugt in einem Bereich von 5 bis 1000 l liegen.
Als Fermentation wird ein in der Industrie häufig durchgeführtes ansatzweises Verfahren (Batch Verfahren) durchgeführt. Weiterhin können beispielsweise auch Fermentationen im Satzbetrieb mit zeitlich variabler (geregelter) oder konstanter Medienzudosierung (Fedbatch) untersucht werden.
Nach Vorlage des Nährmediums im Produktionsreaktor 1 wird dieser mit den entsprechenden Organismen beimpft. Nach Erreichen der Anlauf- (lag-)Phase wird aus dem Produktionsreaktor 1 eine Probe (= Inokulum) entnommen. Dazu wird die gewünschte Menge des Inokulums über eine Leitung 3 in den angeschlossenen Sensor Reaktor 4 steril überführt. Die Entnahme eines Inokulums ist zu beliebig wählbaren Zeitpunkten möglich. Unter Ausnutzung des hydrostatischen Drucks des Produktionsreaktors 1 kann das Inokulum innerhalb von Sekunden (< 1 bis 5 sec) in den Sensor Reaktor 4 überführt werden. Die schnelle Entnahme des Inokulums ist von besonderer Bedeutung, da Änderungen der Kultivierungsbedingungen (des Milieus) für die entnommenen Zellen minimiert werden sollen. Um gleiche Bedingungen im Sensor Reaktor 4 und Produktionsreaktor 1 herzustellen, müssen die Zellen in beiden Reaktoren den gleichen Stoffwechselzustand aufweisen. Die Überführung der Zellen aus dem Produktionsreaktor 1 in den Sensor Reaktor 4 muß so erfolgen, daß keine bzw. nur geringfügige Veränderungen des Stoffwechsels der Zellen stattfinden. Bei den üblicherweise durchgeführten Kontrollfermentationen in einem vom Produktionsreaktor 1 entkoppelten Reaktor, kommt es bei der Entnahme von Zellen aus dem Produktionsreaktor 1 und anschließender Fermentation in einem entkoppelten Reaktor beispielsweise zu Sauerstofflimitierungen oder Substratlimitierungen, so daß sich der Stoffwechselzustand der Zellen im Produktionsreaktor 1 und im entkoppelten Reaktor nicht mehr vergleichen läßt. Die Überführung des Inokulums erfolgt mit so starken zeitlichen Verzögerungen und Veränderungen der Fermentationsbedingungen, daß die Übertragbarkeit der Ergebnisse aus dem entkoppelten Reaktor auf den Produktionsreaktor 1, sehr gering ist.
Bei dem erfindungsgemäßen Verfahren und der Vorrichtung kann die Entnahme des Inokulums über ein Inokulierungssystem erfolgen. Dieses kann aus einer Leitung 3 mit einem ansteuerbarem Ventil 2, z. B. automatisch steuerbares Membranventil oder manuell betriebenes Kugelventil, bestehen. Das Volumen des Inokulums kann beliebig variiert werden und beispielsweise 1 l betragen. Das entnommene Inokulum wird im Sensor Reaktor 4 parallel zum Produktionsreaktor 1 fermentiert. Zur Untersuchung des Fermentationsprozesses im Sensor Reaktor 4 können je nach Bedarf der gesamte Reaktorinhalt oder auch kleinere Probenmengen analysiert werden. Das minimal benötigte Sensor Reaktor Volumen ist von der Anzahl der benötigten Proben sowie der angewandten Analysemethode und der dafür benötigten Zellmasse abhängig. Für eine NMR-Analyse wird beispielsweise eine Zellmasse von ca. 1 g/l benötigt.
Der Sensor Reaktor 4 ist in einer bevorzugten Ausführungsform mit dem Produktionsreaktor 1 so verbunden, daß zwar eine Probe aus dem Produktionsreaktor 1 in den Sensor Reaktor 4 geleitet werden kann, jedoch keine Substanz aus diesem wieder in den Produktionsreaktor 1 gelangt. So kann keine Beeinflussung der Fermentation im Produktionsreaktor 1 durch die Verbindung mit dem Sensor Reaktor 4 erfolgen. Auch eine Infektion des Produktionsreaktors 1 über diese Leitung 3 ist ausgeschlossen. Diese Leitung 3 kann z. B. durch Dampf auch sterilisiert werden.
Beide Reaktoren werden mit Hilfe bestimmter Fermentationsparameter, wie z. B. pH, Temperatur und Gelöstsauerstoffkonzentration, durch beispielsweise eine on-line Analytik überwacht und gesteuert. Die Steuerung und Überwachung des Fermentationsprofils beider Reaktoren erfolgt innerhalb definierter Toleranzgrenzen der gewählten Fermentationsparameter. Die notwendigen Korrekturschritte in der Fermentation eines Reaktors oder beider Reaktoren können durch eine Prozeßüberwachung 5, die Steuerungs- und Regelungseinheiten beinhaltet, durchgeführt werden. Zur On-line und Off-line Beurteilung des Fermentationsprofils können neben heuristischen Methoden z. B. auch Neuronale Netze, Fuzzy-Logic, Hauptkomponentenanalyse oder andere statistische Werkzeuge eingesetzt werden.

Nach Entnahme des Inokulums aus dem Produktionsreaktör 1 und Überführung in den Sensor Reaktor 4 sind die Reaktoren zwar physikalisch getrennt jedoch regelungstechnisch gekoppelt. Der kleinere Sensor Reaktor 4 wird so betrieben, daß er den Fermentationsprozeß des größeren Produktionsreaktors 1 nachbildet. Durch Zugabe von beispielsweise chemischen Substanzen in den Sensor Reaktor 4 kann dann der Einfluss und die Wirkung dieser Substanzen auf den Produktionsreaktor 1 übertragen werden ohne auch im großtechnischen Maßstab im Produktionsreaktor selbst diesen Versuch durchführen zu müssen. Die Zugabe von beispielsweise isotopisch markierten stabilen und instabilen Substanzen wie z. B. ¹³C-markierte Glukose kann über Zugabesysteme 6 und 7 erfolgen. Diese Zugabesysteme 6 und 7 können über elektromagnetischen oder druckluftgesteuerten Ventilen verfügen. Über die Leitung 7 kann die Zufuhr der für die Fermentation benötigten Substanzen aber auch die Zufuhr von markierten Substanzen erfolgen. Über die Leitung 6 kann vorzugsweise eine gepulste Zugabe von Substanzen, insbesondere markierter Substanzen erfolgen. Zur Untersuchung des metabolischen Stoffflusses können auch mit ¹⁵N, ³³S, ¹⁸O oder ²H markierte Substanzen eingesetzt werden. Dabei kann beispielsweise eine Pulsmarkierung z. B. durch eine schnelle druckunterstützte Zugabe der zu verwendenden Markierungssubstanz erfolgen oder die unmarkierte Substanz vollständig oder in einem bestimmten Mischungsverhältnis durch die markierte Substanz ersetzt werden. Bei den Markierungsmessungen kann zwischen der Dokumentation einer quasi-stationären Markierungsanreicherung im Zellmetabolom (Gesamtheit aller Zellmetabolite) oder Zellproteom (Gesamtheit aller Zellproteine) und zwischen der Dokumentation einer dynamischen Markierungsanreicherung im Zellmetabolom unterschieden werden. Bei der quasi-stationären Markierungsanreicherung wird nach Einstellen eines stabilen Markierungsmusters der intrazellulären freien Metabolite bzw. Proteine die Fermentation gestoppt und ein Teil oder der gesamte Inhalt des Sensor Reaktors 4 z. B. für NMR- bzw. massenspektroskopische Analysen eingesetzt. Bei der dynamischen Markierungsanreicherung kann sofort nach Zugabe der Markierungssubstanz in den Sensor Reaktor 4 mit Hilfe z. B. eines schnellen Probenahmesystems 11 und 12 eine Probe aus dem Sensor Reaktor 4 entnommen werden. Diese wird drastisch gekühlt (z. B. Kälteschock in Verbindung mit Lösungsmittel), um den Stoffwechsel direkt zu unterbrechen und später eingefroren. Während des Fermentationsverlaufs werden kontinuierlich in definierten Abständen Proben entnommen und entsprechend wie oben beschrieben behandelt. So kann mit Hilfe dieser zu unterschiedlichen Zeitpunkten des Stoffwechsels gewonnenen Ergebnisse eine Aussage über die Stoffwechseldynamik z. B. über den Stoffwechselweg eines bestimmten Stoffes getroffen werden.
Nach Beendigung der Markierungsexperimente kann eine sterile Reinigung des Sensor Reaktors 4 z. B. mit sterilfiltriertem Wasser, Wasserdampf oder Chemikalien und eine erneute Inokulierung aus dem angeschlossenen Produktionsreaktor 1 erfolgen, so daß während der Fermentation im Produktionsreaktor 1 mehrmals ein Markierungsexperiment oder andere Untersuchungen im Sensor Reaktor 4 durchgeführt werden können. Es ist weiterhin möglich an einen Produktionsreaktor 1 mehrere Sensor Reaktoren 4, z. B. 2 bis 8, anzuschließen, so daß unterschiedliche Untersuchungen parallel nebeneinander durchgeführt werden können.
Die aus dem Sensor Reaktor 4 gewonnenen Ergebnisse können,zur Charakterisierung des Prozesses im technischen Produktionsreaktor 1 herangezogen werden.
Über die aus dem Sensor Reaktor 4 gewonnenen Ergebnisse kann eine Regelung des Stoffwechsels im Produktionsreaktor 1 erfolgen. Der Sensor Reaktor 4 dient dann als Signalgeber für eine On-line Regelung des Produktionsreaktors 1.

### Ausführungsbeispiel:

Zur Untersuchung des Fermentationsprofils eines 300 1 gerührten Produktionsreaktors 1 wurde ein gerührter Sensor Reaktor 4 mit einem Füllvolumen von einem Liter über eine Leitung 3 angeschlossen. Es wurde ein ansatzweises (Batch) Verfahren gewählt. Dazu wurde nach Vorlage eines optimierten Nährmediums mit Glukose als Hauptkohlenstoffquelle (Weuster-Botz D., Kelle R., Frantzen M., Wandrey C.: "Substrate controlled fed-batch production of L-lysine with *Corynebacterium glutamicum*"; Biotechnol. Prog. (1997); 13, 387-393) im 300 1 Fermenter, Beimpfen mit dem Aminosäureproduzenten *Corynebacterium glutamicum* MH20-22b (L-Leucin auxotroph, feedback-resistente Aspartatkinase) (Schrumpf b., Eggeling L., Sahm H.: "Isolation and prominent characteristics of an L-Lysine hyperproducing strain of *Corynebacterim glutamicum*"*;* App. Microbiol. Biotechnol. (1992); 37, 566-571) und Fermentation bis kurz vor Ende der lag-Phase ein Inokulum über die Leitung 3 in den an diesen angeschlossenen Sensor Reaktor 4 innerhalb von 4 Sekunden unter zu Hilfenahme des Druckunterschiedes (auf Grund des hydrostatischen Drucks) von 0,4 bar steril überführt. Die aktuellen Prozessparameter wie Wasserstoffionenkonzentration (pH Sollwert = 7.0), Temperatur (Sollwert = 30 °C) und Gelöstsauerstoffkonzentration (pO₂ Sollwert = 30%) wurden im 300 l Fermenter on-line gemessen und dienten als Sollwerte für die Regelung des Sensor Reaktors 4. Zur Beurteilung der Vergleichbarkeit der Fermentationsverläufe im 300 l Produktionsreaktor 1 und im Sensor Reaktor wurden simultan in Zeitabschnitten von 30 Minuten Proben aus dem Sensor Reaktor 4 und dem 300 l Produktionsreaktor 1 entnommen. Die Proben wurden zentrifugiert und der Überstand für spätere Analysen bei -20°C eingefroren. Im Medienüberstand der Proben wurden mittels HPLC-Analytik die Konzentrationen von organischen Säuren und Aminosäuren quantifiziert. Weiterhin wurde die Biomassenkonzentration mittels Optische Dichtemessung bei 600 nm bestimmt. Nach einer Fermentationsdauer von 2 Stunden wurde der Sensor Reaktor 4 vollständig entleert und zweimal mit sterilfiltriertem Wasser gereinigt. Anschließend wurde zu Beginn der exponentiellen Wachstumsphase der Hauptfermentation im 300 l Produktionsreaktor 1 erneut ein Inokulum aus diesem in den Sensor Reaktor 4 überführt und der oben beschriebene Vorgang wiederholt.

Nach Auswertung der Versuchsergebnisse zeigte sich, daß durch die gekoppelte online Regelung eine weitgehend synchrone Fermentation im 300 l Produktionsreaktor 1 und im angeschlossenen Sensor Reaktor 4 erreicht werden konnte. Sowohl die Biomassekonzentration (Fig. 2) als auch die Konzentrationen von L-Lysin, L-leucin, Glycin und Oxoglutarat (Fig. 3-6) stimmten zwischen beiden Reaktoren so gut überein, daß der Korrelationskoeffizient immer nahezu 1 betrug. Daraus kann auch eine hohe Korrelation des Stoffwechselzustandes der Zellen in beiden Reaktoren geschlossen werden. Ergebnisse aus dem Sensor Reaktor 4 können so direkt auf den Produktionsreaktor übertragen werden. Weiterhin konnte gezeigt werden, dass zu frei bestimmbaren Zeitpunkten das erfindungsgemäße Verfahren mehrmals während der Hauptfermentation mit Hilfe des angeschlossenen Sensor Reaktors 4 wiederholt werden kann.

## Patentansprüche

1. Verfahren zur Untersuchung von Fermentationsprozessen, bei dem ein Fermentationsprozess in einem Produktionsreaktor durchgeführt wird,
**dadurch gekennzeichnet,**
**dass** ein Inokulum aus dem Fermentationsprozess aus dem Produktionsreaktor (1) in einem Reaktor (4) überführt wird, welcher ein Größenverhältnis zum Produktionsreaktor (1) von ≤ 1:10 besitzt, der Reaktor (4) vom Produktionsreaktor (1) physikalisch getrennt wird, und dass der Reaktor (4) über eine Prozessüberwachung (5) so gesteuert wird, dass die aktuellen Prozessparameter des Reaktors (4) mit denen des Produktionsreaktors (1) übereinstimmen und dass ein Probenahmesystem (11 und 12) eingesetzt wird.

2. Verfahren nach Anspruch 1,
**dadurch gekennzeichnet,**
**dass** das Inokulum aus dem Produktionsreaktor (1) innerhalb von < 1 bis 5 sec in die Sensor Reaktoren (4) überführt wird.

3. Verfahren nach einem der Ansprüche 1 oder 2,
**dadurch gekennzeichnet,**
**dass** Zugabesysteme (6 und 7) eingesetzt werden.

4. Verfahren nach einem der Ansprüche 1 bis 3,
**dadurch gekennzeichnet,**
**dass** eine Batch- oder Fed-Batch Fermentation durchgeführt wird.

5. Verfahren nach einem der Ansprüche 1 bis 4,
**dadurch gekennzeichnet,**
**dass** Proben aus dem Produktionsreaktor (1) steril in die Sensor Reaktoren (4) überführt werden.

6. Verfahren nach einem der Ansprüche 1 bis 5,
**dadurch gekennzeichnet,**
**dass** Substanzen, die mit Isotopen markiert sind, den Sensor Reaktoren (4) zugesetzt werden.

7. Verfahren nach Anspruch 6,
**dadurch gekennzeichnet,**
**dass** Substanzen, die mit ¹³C, ¹⁵N , ³³S, ¹⁸O oder ²H markiert sind den Sensor Reaktoren (4) zugesetzt werden.

8. Verfahren nach einem der Ansprüche 6 bis 7,
**dadurch gekennzeichnet,**
**dass** eine Pulsmarkierung durchgeführt wird.

9. Verfahren nach einem der Ansprüche 6 bis 8,
**dadurch gekennzeichnet,**
**dass** die unmarkierte Substanz vollständig oder teilweise durch isotopisch markierte Substanz ersetzt wird.

10. Verfahren nach einem der Ansprüche 1 bis 9,
**dadurch gekennzeichnet,**
**dass** ein Teil oder der gesamte Reaktorinhalt der Sensor Reaktoren (4) zur Analyse entnommen wird.

11. Verfahren nach einem der Ansprüche 1 bis 10,
**dadurch gekennzeichnet,**
**dass** die durch Markierung erhaltene Information zur Steuerung des Produktionsreaktors (1) eingesetzt wird.

12. Verfahren nach einem der Ansprüche 1 bis 11,
**dadurch gekennzeichnet,**
**dass** ein Inokulum zu unterschiedlichen Zeitpunkten aus dem Produktionsreaktor (1) in den Sensor Reaktor (4) überführt wird.

13. Verfahren nach einem der Ansprüche 1 bis 12,
**dadurch gekennzeichnet,**
**dass** die Leitungen zwischen Sensor Reaktoren (4) und Produktionsreaktor (1) sterilisiert werden.

14. Verfahren nach einem der Ansprüche 1 bis 13,
**dadurch gekennzeichnet,**
**dass** die Sensor Reaktoren (4) wiederholt befüllt werden.

15. Vorrichtung zur Durchführung des Verfahrens nach einem der Ansprüche 1 bis 14,
**gekennzeichnet durch** folgende Merkmale
- einen Produktionsreaktor (1)
- einen Reaktor (4), welcher mit dem Produktionsreaktor (1) über eine Leitung (3) mit einem Ventil (2) in Verbindung steht,
- eine Prozessüberwachung (5), welche mittels Mess- und Regelsystem (10) bewirkt, dass die aktuellen Prozessparameter des Reaktors (4) mit denen des Produktionsreaktors (1) übereinstimmen,
- der Reaktor (4) steht zum Produktionsreaktor (1) in einem Größenverhältnis von ≤ 1:10,
- der Reaktor (4) umfasst eine Leitung mit Probenahmeventil (11).

16. Vorrichtung nach Anspruch 15,
**dadurch gekennzeichnet,**
**dass** sie ein Inokulierungssystem umfasst.

17. Vorrichtung nach einem der Ansprüche 15 oder 16,
**dadurch gekennzeichnet,**
**dass** sie ein Probenahmesystem (11 und 12) umfasst.

18. Vorrichtung nach einem der Ansprüche 15 bis 17,
**dadurch gekennzeichnet,**
**dass** sie Zugabesysteme (6 und 7) umfasst.

19. Vorrichtung nach einem der Ansprüche 15 bis 18,
**dadurch gekennzeichnet,**
**dass** die Leitungen (3) zwischen Sensor Reaktoren (4) und Produktionsreaktor(1) sterilisierbar sind.

## Claims

1. Method for investigation of fermentation processes, in which a fermentation process is carried out in a production reactor,
**characterised in**
**that** an inoculum from the fermentation process is transferred from the production reactor (1) to a reactor (4) with a proportion of ≤1:10 to the production reactor (1), the reactor (4) is physically separated from the production reactor (1) and the reactor (4) is controlled by process monitoring (5) so that the current process parameters of the reactor (4) agree with those of the production reactor (1) and a sampling system (11 and 12) is used.

2. Method according to claim 1,
**characterised in**
**that** the inoculum is transferred from the production reactor (1) to the sensor reactors (4) within < 1 to 5 seconds.

3. Method according to claims 1 or 2,
**characterised in**
**that** feed systems (6 and 7) are used.

4. Method according to one of claims 1 to 3,
**characterised in**
**that** batch or fed batch fermentation is carried out.

5. Method according to claims 1 to 4,
**characterised in**
**that** samples are transferred from the production reactor (1) to the sensor reactors (4) in a sterile way.

6. Method according to claims 1 to 5,
**characterised in**
**that** substances marked with isotopes are added to the sensor reactors (4).

7. Method according to claim 6,
**characterised in**
**that** substances marked with ¹³C, ¹⁵N, ³³S, ¹⁸O or ²H are added to the sensor reactors (4).

8. Method according to claims 6 to 7,
**characterised in**
**that** pulse marking is carried out.

9. Method according to one of claims 6 to 8,
**characterised in**
**that** the unmarked substance is completely or partly replaced by substance marked with isotopes.

10. Method according to one of claims 1 to 9,
**characterised in**
**that** a part or the whole of the reactor content of the sensor reactors (4) is removed for analysis.

11. Method according to one of claims 1 to 10,
**characterised in**
**that** the information obtained by marking is used to control the production reactor (1).

12. Method according to one of claims 1 to 11,
**characterised in**
**that** an inoculum is transferred from the production reactor (1) to the sensor reactors (4) at various times.

13. Method according to one of claims 1 to 12,
**characterised in**
**that** the lines between sensor reactors (4) and production reactor (1) are sterilised.

14. Method according to one of claims 1 to 13,
**characterised in**
**that** the sensor reactors (4) are filled repeatedly.

15. Device to cany out the method according to one of claims 1 to 14,
**characterised by** the following characteristics
- a production reactor (1),
- a reactor (4), which is connected to the production reactor (1) through a line (3) with a valve (2),
- process monitoring (5), which makes the current process parameters of the reactor (4) agree with those of the production reactor (1) by means of a measuring and regulating system (10),
- the reactor (4) has a proportion of ≤1:10 to the production reactor (1),
- the reactor (4) has a line with a sampling valve (11).

16. Device according to claim 15,
**characterised in**
**that** it has an inoculating system.

17. Device according to one of claims 15 or 16,
**characterised in**
**that** it has a sampling system (11 and 12).

18. Device according to one of claims 15 to 17,
**characterised in**
**that** it has feed systems (6 and 7).

19. Device according to one of claims 15 to 18,
**characterised in that** the lines (3) between sensor reactors (4) and production reactor (1) can be sterilised.

## Revendications

1. Procédé d'analyse de processus de fermentation, dans lequel un processus de fermentation est réalisé dans un réacteur de production,
**caractérisé en ce qu'**un inoculat provenant du processus de fermentation est transféré du réacteur de production (1) dans un réacteur (4), lequel possède un rapport dimensionnel par rapport au réacteur de production (1) ≤ 1:10, le réacteur (4) étant séparé physiquement du réacteur de production (1), et **en ce que** le réacteur (4) est commandé par une surveillance de processus (5), de sorte que les paramètres de processus actuels du réacteur (4) coïncident avec ceux du réacteur de production (1) et **en ce qu'**on utilise un système d'échantillonnage (11 et 12).

2. Procédé selon la revendication 1,
**caractérisé en ce que**
l'inoculat est transféré du réacteur de production (1) en moins de 1 à 5 s dans les réacteurs de détecteur (4).

3. Procédé selon l'une quelconque des revendications 1 ou 2,
**caractérisé en ce qu'**on utilise des systèmes d'addition (6 et 7).

4. Procédé selon l'une quelconque des revendications 1 à 3,
**caractérisé en ce qu'**on réalise une fermentation classique ou à écoulement discontinu.

5. Procédé selon l'une quelconque des revendications 1 à 4,
**caractérisé en ce que** les échantillons sont transférés de façon stérile du réacteur de production (1) aux réacteurs de détection (4).

6. Procédé selon l'une quelconque des revendications 1 à 5,
**caractérisé en ce que** des substances, qui sont marquées par des isotopes, sont ajoutées aux réacteurs de détection (4).

7. Procédé selon la revendication 6,
**caractérisé en ce que**
des substances, qui sont marquées par ¹³C, ¹⁵N, ³³S, ¹⁸O ou ²H, sont ajoutées aux réacteurs de détection (4).

8. Procédé selon l'une quelconque des revendications 6 à 7,
**caractérisé en ce qu'**on réalise un marquage à impulsions.

9. Procédé selon l'une quelconque des revendications 6 à 8,
**caractérisé en ce que** la substance non marquée est remplacée entièrement ou en partie par une substance marquée de façon isotopique.

10. Procédé selon l'une quelconque des revendications 1 à 9,
**caractérisé en ce qu'**une partie ou l'ensemble du contenu du réacteur des réacteurs de détection (4) est prélevé(e) pour être analysé(e).

11. Procédé selon l'une quelconque des revendications 1 à 10,
**caractérisé en ce que** l'information reçue par le marquage est utilisée pour commander le réacteur de production (1).

12. Procédé selon l'une quelconque des revendications 1 à 11,
**caractérisé en ce qu'**un inoculat, à différents moments, est transféré du réacteur de production (1) au réacteur de détection (4).

13. Procédé selon l'une quelconque des revendications 1 à 12,
**caractérisé en ce que** les conduites entre les réacteurs de détection (4) et le réacteur de production (1) sont stérilisées.

14. Procédé selon l'une quelconque des revendications 1 à 13,
**caractérisé en ce que**
les réacteurs de détection (4) sont à nouveau remplis.

15. Dispositif destiné à réaliser le procédé selon l'une quelconque des revendications 1 à 14,
**caractérisé par** les caractéristiques suivantes
- un réacteur de production (1)
- un réacteur (4), lequel est relié avec le réacteur de production (1) par l'intermédiaire d'une conduite (3) munie d'une soupape (2),
- une surveillance de processus (5), laquelle entraîne, au moyen d'un système de mesure et de réglage (10), que les paramètres de processus actuels du réacteur (4) coïncident avec ceux du réacteur de production (1),
- le réacteur (4) se situe par rapport au réacteur de production (1) dans un rapport dimensionnel de ≤ 1:10,
- le réacteur (4) comporte une conduite dotée d'une soupape d'échantillonnage (11).

16. Dispositif selon la revendication 15,
**caractérisé en ce qu'**il comporte un système d'inoculation.

17. Dispositif selon l'une quelconque des revendications 15 ou 16,
**caractérisé en ce qu'**il comporte un système d'échantillonnage (11 et 12).

18. Dispositif selon l'une quelconque des revendications 15 à 17,
**caractérisé en ce qu'**il comporte des systèmes d'addition (6 et 7).

19. Dispositif selon l'une quelconque des revendications 15 à 18,
**caractérisé en ce que**
les conduites (3) entre les réacteurs de détection (4) et le réacteur de production (1) sont stérilisables.
